# EUROPEAN PATENT APPLICATION

(11) **EP 4 424 336 A1**
(43) Date of publication of application: **04.09.2024**
(21) Application number: 22884939.4
(22) Date of filing: 14.03.2022
(51) Int. Cl.: A61M 1/00, A61M 27/00, A61M 31/00, A61B 10/00

(54) **IMPLANTABLE LIQUID TRANSFER DEVICE AND LIQUID TRANSFER CONTROL SYSTEM**

(30) Priority: 27.10.2021 CN 202111258527
(71) Applicant: Institute of Flexible Electronics Technology of Thu, Zhejiang, Jiaxing, Zhejiang 314006 (CN); Shanghai GE Ruimeng Medtech Inc, Shanghai 201210 (CN); Tsinghua University, Haidian District, Beijing 100084 (CN)
(72) Inventor: FENG, Xue, Beijing 100084 (CN); ZENG, Jin, Jiaxing, Zhejiang 314006 (CN); FU, Ji, Jiaxing, Zhejiang 314006 (CN); WANG, Zhiyong, Shanghai 201210 (CN)
(74) Representative: advotec.
(86) International application number: PCT/CN2022/080798
(87) International publication number: WO 2023/071022

(57) **Abstract**

An implantable liquid transfer device and a liquid transfer control system are provided. The device includes a pump body (11), a control valve (101), a liquid inlet pipe (15) and a liquid outlet pipe (16), wherein the control valve (101) includes a first control valve (13) and a second control valve (14), and one end of the liquid inlet pipe (15) is connected with one end of the pump body (15), and one end of the liquid outlet pipe (16) is connected with the other end of the pump body (11), and the pump body (11) is configured to drive body fluid to flow from the liquid inlet pipe (15) to the liquid outlet pipe (16), and the other end of the liquid outlet pipe (16) is provided with a plurality of branch pipes (160); and the first control valve (13) is provided on the liquid inlet pipe (15); and the second control valve (14) is provided on the liquid outlet pipe, and the second control valve (14) is a multi-way valve and is configured to control the liquid outlet pipe (16) to be communicated with the branch pipes (160), and the plurality of the branch pipes (160) are respectively provided at different tissues. The implantable liquid transfer device avoids infection by repeated punctures and has good universality for ascites patients. Moreover, the design of the multi-branch pipe (160) can be used for transferring liquids, administrating drugs or taking samples for testing, and draining the liquids which are formed by such as malignancy of a tumor and containing inflammatory factors to the outside.

## Description

### CROSS-REFERENCE TO RELATED APPLICATIONS

This application claims priority of Chinese patent application No. 202111258527.0, filed on October 27, 2021, and titled "implantable liquid transfer device and liquid transfer control system", the entire contents of which are hereby incorporated by reference.

### FIELD

The present application relates to the technical field of medical devices, in particular to an implantable liquid transfer device and a liquid transfer control system.

### BACKGROUND

Edema is a pathological process in which excessive body fluids accumulate in tissue spaces or body cavities, such as a cardiac edema induced by a heart failure, a renal edema caused by a primary renal disease, a hepatic edema caused by abnormal accumulation of body fluids induced by a liver disease, a pulmonary edema induced by excessive body fluids accumulated in an interstitial lung and/or overflowing into an alveoli, and a cerebral edema caused by the increase of fluid content in brain tissue and the increase of brain volume. Main causes of a tissue edema include an increased capillary blood pressure, a decreased plasma colloid osmotic pressure, an increased capillary permeability and a blocked lymphatic reflux. Tissue edema will cause certain organ dysfunction, for example, a gastrointestinal mucosal edema can affect digestion and absorption, a pulmonary edema can cause respiratory dysfunction, pericardial hydrops can affect the heart's pumping function, a laryngeal edema can cause airway obstruction and even suffocation, and a brain edema can cause an increased intracranial pressure and even form cerebral hernia, which is life-threatening.

At present, main clinical treatments for tissue edema include giving diuretics and other drugs to strengthen body metabolism and treat edema. In view of the intractable edema that is ineffective in drug treatment, it is necessary to drain the accumulated liquid, implant the drainage pipe under the skin, make a fistula, and drain the liquid to the outside of the body regularly. This method has a great risk of infection. Also, drainage channels can be built in the body and drainage pipes with valves are implanted to drain cerebrospinal fluid to the abdominal cavity. However, such products have limited application scope and provide limited liquid transfer, which cannot be used in other tissue edema. Based on the realization of more transfer scenarios and better control, the liquid transfer is changed from passive transfer to active transfer, and an active micro-pump is implanted to form a transfer channel in the body. However, the present application of active devices leads to the incompatibility of MR (magnetic resonance), and the blockage of pump body and pipeline is easy to cause product failure, thus the product safety needs to be improved. Moreover, when body fluids are transferred to the body, which forms a transfer channel in the body, the blockage of the pipeline will cause the function of the transfer system to fail. Frequent puncture will cause inflammation and infection, and seriously reduce the living standards of patients.

### Technical problems

Clinically, according to different transfer scenarios and different development stages of the same disease, the properties of the transferred liquid will be different. For example, the transfer of large particles and viscous substances has higher requirements for the pump body and pipeline. Moreover, the existing transfer products have limited service life due to having electronic products such as a power supply, and also have certain restrictions on clinical MR (magnetic resonance) examination, which affects the physiological health of patients and the diagnosis and treatment of diseases.

### SUMMARY

In order to overcome the shortcomings and deficiencies in the related art, the purpose of the present application is to provide an implantable liquid transfer device and a liquid transfer control system, so as to solve the problems that the liquid transfer device in the related art requires frequent punctures and is difficult to sample and inject drugs.

The purpose of the present application is achieved by the following technical solution.

The present application provides an implantable liquid transfer device, which includes a pump body, a control valve, a liquid inlet pipe and a liquid outlet pipe, wherein the control valve includes a first control valve and a second control valve; one end of the liquid inlet pipe is connected with one end of the pump body; one end of the liquid outlet pipe is connected with the other end of the pump body; the pump body is configured to drive body fluids to flow from the liquid inlet pipe to the liquid outlet pipe; the other end of the liquid outlet pipe is provided with a plurality of branch pipes; and the first control valve is provided on the liquid inlet pipe; and the second control valve is provided on the liquid outlet pipe, is a multi-way valve and is configured to control the liquid outlet pipe to be communicated with the branch pipes, and the plurality of branch pipes are respectively provided at different tissues.

Further, the pump body is a positive displacement pump, which includes a pump housing and a magnetic plate connected with the pump housing, wherein the pump housing has a cavity, the magnetic plate is provided in the pump housing and divides the cavity into two chambers, the pump housing is provided with two first ports communicated with one of the chambers, and the magnetic plate can move or deform in a magnetic field and change the volumes of the two chambers.

Further, an edge of the magnetic plate is provided with a clamping block, and the magnetic plate is clamped with the pump housing through the clamping block.

Further, the two first ports are detachably connected with the liquid inlet pipe and the liquid outlet pipe respectively.

Further, the implantable liquid transfer device also includes a capsule, one end of the liquid inlet pipe far away from the pump body is connected with the capsule, and the capsule is provided with a plurality of through holes.

Further, a semi-permeable membrane is provided at the through hole of the capsule, and body fluids can pass through the semi-permeable membrane and enter the capsule.

Further, the capsule has a plurality of branch structures, each branch structure is respectively provided in a different edema area, each branch structure is provided with a plurality of through holes, and each branch structure is communicated with the liquid inlet pipe.

Further, the first control valve is a magnetic control valve, and the first control valve includes a first valve housing, a first flexible pipe and a first magnetic rotation member, wherein the first valve housing has a first accommodating cavity and two second ports communicated with the first flexible pipe; the first magnetic rotation member is eccentrically provided in the first accommodating cavity and can rotate in the first accommodating cavity; and the first magnetic rotation member can rotate in a magnetic field and adjust the flow of the first flexible pipe.

Further, one end of the liquid outlet pipe far away from the pump body is provided with two branch pipes, namely a first branch pipe and a second branch pipe respectively, and the control valve also includes a one-way valve, wherein the first branch pipe is provided with the one-way valve and a fixing device, and the fixing device is provided at one end of the first branch pipe far away from the liquid outlet pipe; and the second branch pipe is provided with a fixing point.

Further, the second control valve is a magnetic control valve, and includes a second valve housing and a second magnetic rotation member, wherein the second valve housing has a second accommodating cavity and three third ports communicating with the second accommodating cavity, and the three third ports are respectively communicated with the liquid outlet pipe, the first branch pipe and the second branch pipe, and the second magnetic rotation member is provided in the second accommodating cavity and forms a liquid guide channel with an inner wall of the second valve housing; and the second magnetic rotation member can rotate in the second accommodating cavity, and includes a rotation part and a blocking part which are connected with each other, and can rotate in a magnetic field and enable the blocking part to selectively block one of the third ports.

Further, the one-way valve is a diaphragm valve, and the diaphragm valve includes a valve pipe and a plurality of magnetic membrane flaps provided in the valve pipe, wherein the magnetic membrane flaps can be unfolded or rolled up in a magnetic field; and in an initial state, the magnetic membrane flaps curl in the valve pipe and block the conduction of the valve pipe; and in an infusion state, the magnetic membrane flaps are unfolded and curled at intervals in an infusion direction.

Further, the fixing device includes an injection port, a control pipe and a plurality of microbubble structures, wherein the injection port is communicated with the plurality of microbubble structures through the control pipe, and the microbubble structures are made of elastic materials.

Further, the implantable liquid transfer device further includes a data monitoring component for monitoring body fluid information, and the data monitoring component includes at least one of a pressure sensor, a flow sensor or an inflammatory factor sensor, wherein the pressure sensor is configured to detect a hydraulic pressure of the edema area, the flow sensor is configured to detect the flow of the liquid transferred by the pump body, and the inflammatory factor sensor is configured to detect the inflammatory condition of the edema area.

The present application also provides a liquid transfer control system, which includes a transfer control device and the implantable liquid transfer device as mentioned above, wherein the transfer control device is configured to control the implantable liquid transfer device to operate normally.

Further, the transfer control device includes a power supply, a processor, a driving module and a data receiving module, wherein the power supply, the driving module and the data receiving module are all electrically coupled with the processor; the implantable liquid transfer device includes a data monitoring component, the driving module is configured to drive the pump body, the control valve and the data monitoring component to operate, the data monitoring component is configured to monitor body fluid information, the data receiving module is configured to receive the body fluid information, and the processor controls the operation state of the driving module to drive the pump body and the control valve according to the body fluid information.

Further, the transfer control device further includes a communication module, and the communication module is configured to send the body fluid information to the mobile terminal.

### Beneficial effects

By providing the pump body in an implantable liquid transfer device, the pump body is implanted in the body together with the implantable liquid transfer device, so that the liquid can be actively transferred to the natural cavity to be discharged, the balance of local tissue pressure is maintained, the infection caused by repeated puncture is avoided, and the pump body has good universality for ascites patients. The design of the plurality of branch pipes can be used to transfer liquids, administer drugs or take samples for testing, and drain the liquids which are formed by such as malignancy of a tumor and containing inflammatory factors to the outside of the body, so as to realize the multifunctional transfer of liquid management.

### BRIEF DESCRIPTION OF THE DRAWINGS

Fig. 1 is a schematic structural view of an implantable liquid transfer device in the present application;
Fig. 2 is a schematic structural view of a pump body in the present application;
Fig. 3 is a schematic structural view of a first control valve in the present application;
Fig. 4 is a schematic structural view of a second control valve in the present application;
Fig. 5 is a first schematic structural view of a capsule in the present application;
Fig. 6 is a second schematic structural view of a capsule in the present application;
Fig. 7 is a schematic structural view of a fixing device in the present application;
Fig. 8 is a schematic structural view of a one-way valve in the present application;
Figs. 9a-9d are schematic structural diagrams of the working principle of the one-way valve in the present application;
Fig. 10 is a structural block diagram of a liquid transfer control system in the present application.

### DETAILED DESCRIPTION

In order to further illustrate the technical means and efficacy adopted by the present application to achieve the intended application purpose, the specific implementation, structure, characteristics and efficacy of an implantable liquid transfer device and a liquid transfer control system proposed according to the present application are described in detail as follows with the attached drawings and preferred embodiments.

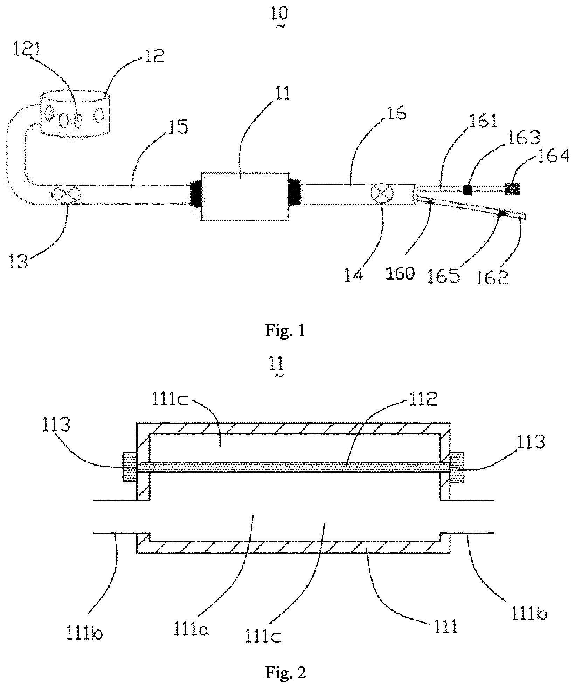

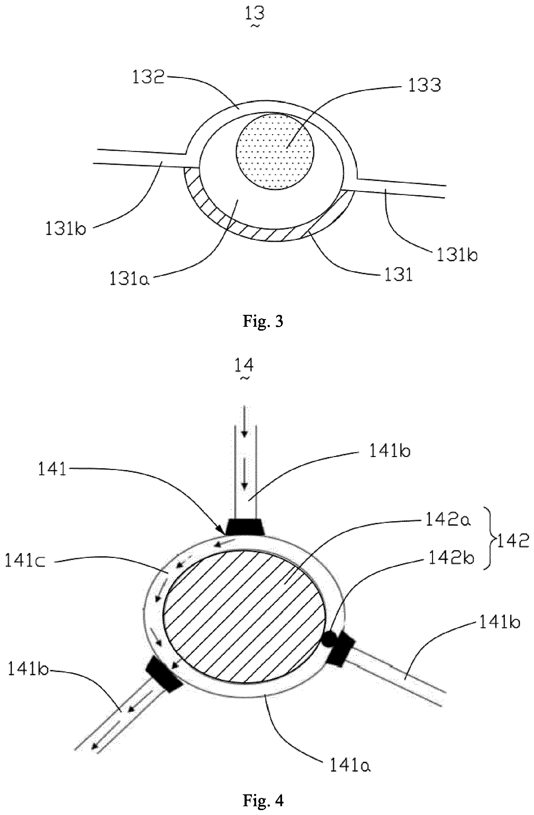

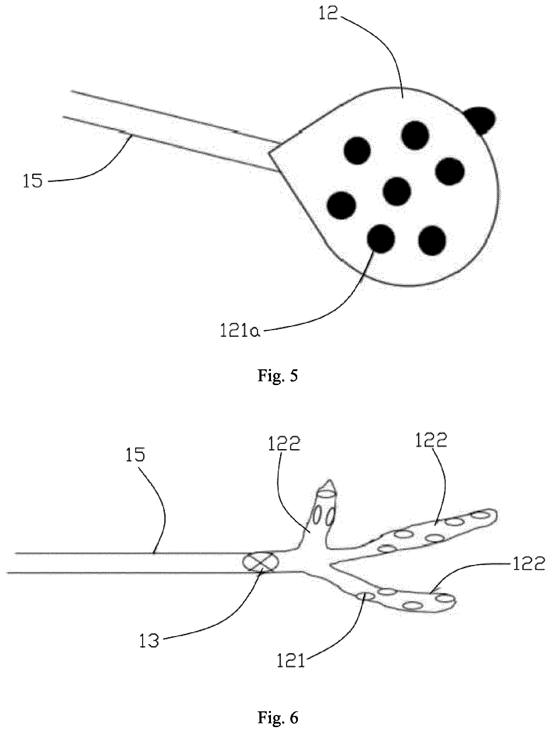

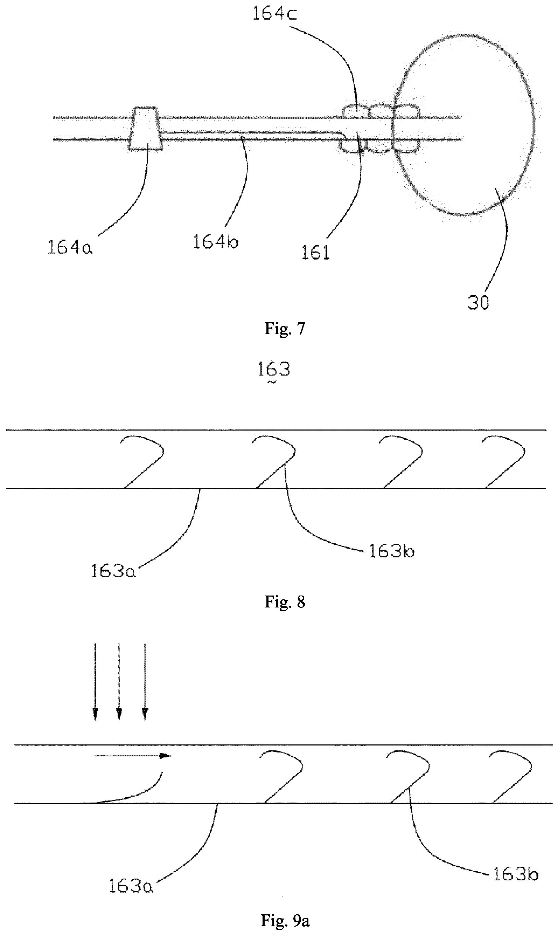

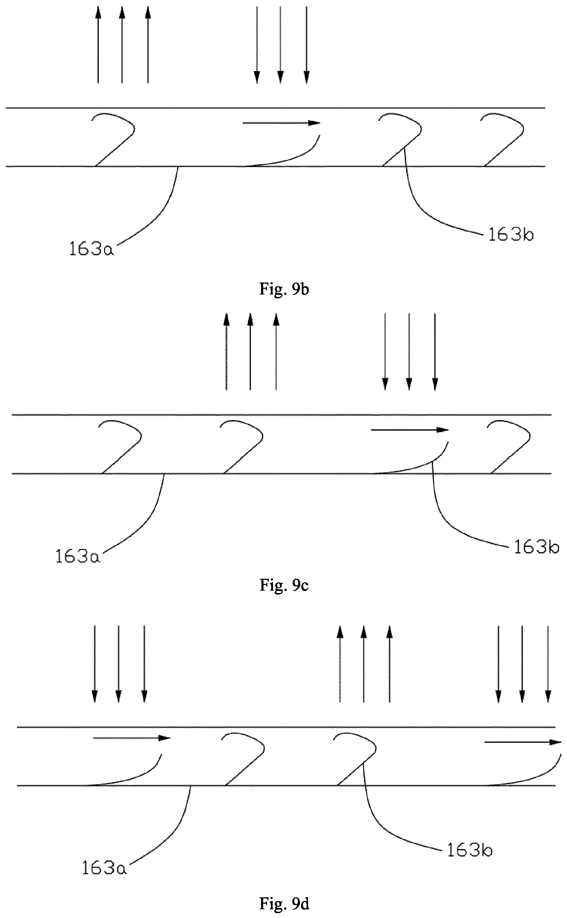

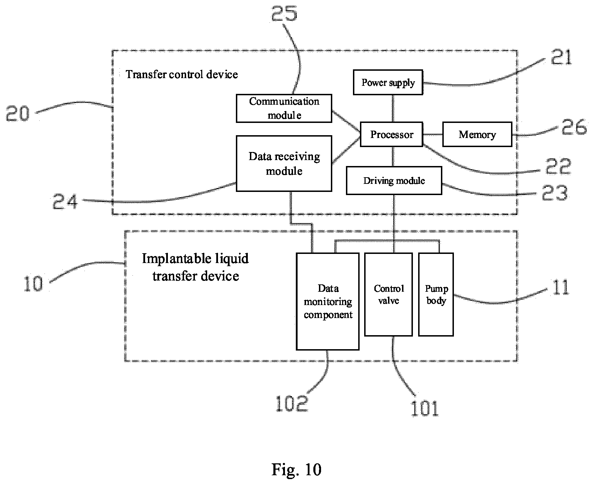

Fig. 1 is a structural schematic diagram of an implantable liquid transfer device in the present application, Fig. 2 is a structural schematic diagram of a pump body in the present application, Fig. 3 is a structural schematic diagram of a first control valve in the present application, Fig. 4 is a structural schematic diagram of a second control valve in the present application, Fig. 5 is a first structural schematic diagram of a capsule in the present application, Fig. 6 is a second structural schematic diagram of a capsule in the present application, Fig. 7 is a structural schematic diagram of a fixing device in the present application, Fig. 8 is a structural schematic diagram of a one-way valve in the present application, and Figs. 9a-9d are schematic structural diagrams of the working principle of a one-way valve in the present application, and Fig. 10 is the structural block diagram of a liquid transfer control system in the present application.

As shown in Fig. 1 to Fig. 8 and Fig. 10, an implantable liquid transfer device provided by the present application includes a pump body 11, a control valve 101, a liquid inlet pipe 15 and a liquid outlet pipe 16, and the control valve 101 includes a first control valve 13 and a second control valve 14, one end of the liquid inlet pipe 15 is connected with one end of the pump body 11, and one end of the liquid outlet pipe 16 is connected with the other end of the pump body 11, and the pump body 11 is used for driving body fluids to flow from the liquid inlet pipe 15 to the liquid outlet pipe 16. The other end of the outlet pipe 16 is provided with a plurality of branch pipes 160. The first control valve 13 is provided on the inlet pipe 15, and the second control valve 14 is provided on the outlet pipe 16. The second control valve 14 is a multi-way valve and is used for controlling the outlet pipe 16 to communicate with the branch pipes 160, and the plurality of branch pipes 160 are respectively provided at different tissues.

In the present application, by providing the pump body 11 in an implantable liquid transfer device, the pump body 11 is implanted in the body together with the implantable liquid transfer device, so that the liquid can be actively transferred to the natural cavity to be discharged, the balance of local tissue pressure is maintained, the infection caused by repeated punctures is avoided, and the universality for ascites patients is good; The plurality of branch pipes 160 of the liquid outlet pipe 16 can be designed to transfer liquid, administer drugs or take samples for detection, and drain the liquid which are formed by such as malignancy of a tumor and containing inflammatory factors to the outside of the body, so as to realize multifunctional transfer of liquid management.

Further, the inside of the liquid inlet pipe 15 and the liquid outlet pipe 16 contains hydrophilic coating and developing coating, which is convenient for X-ray detection.

In this embodiment, the end of the liquid outlet pipe 16 far away from the pump body 11 is provided with two branch pipes 160, namely a first branch pipe 161 and a second branch pipe 162 respectively, and the control valve 101 also includes a one-way valve 163. The first branch pipe 161 is provided with the one-way valve 163 and a fixing device 164, and the fixing device 164 is provided at the end of the first branch pipe 161 far away from the liquid outlet pipe 16, and the one-way valve 163 is used for preventing liquid from flowing backwards. The fixing device 164 is used to fix the first branch pipe 161 to a tissue, such as a bladder or a vein. The second branch pipe 162 is provided with a fixing point 165, and the fixing point 165 is provided with a rubber structure to facilitate to inject drugs or the detection of liquids sucked out, and the outer surface is provided with a wrapping sleeve made of polyethylene or polyester with good biocompatibility. The fixing point 165 is used to fix the second branch pipe 162 to a subcutaneous tissue. When the second control valve 14 controls the liquid outlet pipe 16 to communicate with the second branch pipe 162, sampling or drug administration can be carried out through the second branch pipe 162. During drug administration, the pump body is closed, the first control valve 13 is opened, and the drug is transferred to the edema area through the second branch pipe 162, the liquid outlet pipe 16, the liquid inlet pipe 15 and the capsule 12. Of course, the end of the liquid outlet pipe 16 far from the pump body 11 can also be provided with more branch pipes to realize multifunctional transfer of liquid management.

Further, the type of the pump body 11 can be one of the positive displacement pumps such as a peristaltic pump, a gear pump, a piezoelectric pump and a diaphragm pump, and the constituent materials of the pump body 11 at least contain flexible materials or memory metals, and the pump body 11 changes in volume under certain driving to generate a driving force. Preferably, the outer surface of the pump body 11 is provided with materials such as silica gel with good biocompatibility to prevent the rejection of the pump body 11 by the human defense system.

In this embodiment, as shown in Fig. 2, the pump body 11 is a diaphragm pump in a positive displacement pump. The diaphragm pump includes a pump housing 111 and a magnetic plate 112 connected with the pump housing 111. The pump housing 111 has a disc-shaped structure and a cavity 111a. The magnetic plate 112 is provided in the pump housing 111 and divides the cavity 111a into two chambers 111c, and the pump housing 111 is provided with two first ports 111b communicating with one of the chambers 111c, and the magnetic plate 112 can move or deform in the magnetic field and change the volumes of the two chambers 111c, so as to control the normal operation of the pump body 11 through the magnetic field outside the body. The two first ports 111b are detachably connected with the liquid inlet pipe 15 and the liquid outlet pipe 16, respectively, so as to facilitate the component of the pump body 11 with the liquid inlet pipe 15 and the liquid outlet pipe 16, and the first ports 111b achieve sealing performance on the premise of ensuring the connection strength, thus forming a liquid transfer path.

An edge of the magnetic plate 112 is provided with a clamping block 113, and the magnetic plate 112 is clamped with the pump housing 111 through the clamping block 113. Of course, the side wall of the pump body 11 is provided with a clamping hole, which is convenient for the magnetic plate 112 to be clamped with the pump housing 111. The clamping block 113 can be made of polymer materials, such as at least two or more materials such as flexible silica gel, polyurethane, polyether, polysulfone, etc., and can also be made of memory metal materials. The magnetic plate 112 is coated to avoid adhesion to the pump housing 111, which clings to but does not adhere to the pump housing 111.

In this embodiment, the magnetic plate 112 is made of flexible magnetic material and can be deformed in a magnetic field, for example, magnetic powder can be added into flexible silica gel. The edge of the magnetic plate 112 can be fixed with the pump housing 111 to increase the sealing performance. Of course, the magnetic plate 112 can be made of hard material, but the edge of the magnetic plate 112 is movably connected with the pump housing 111 to change the volumes of the two chambers 111c through the movement of the magnetic plate 112.

The pump body 11 is implanted in superficial layer of subcutaneous tissue, and the magnetic plate 112 can be used for normal liquid transfer. However, when patients need to do MR examination, the magnetic plate 112 in the pump body 11 can be taken out, and after the examination is completed, the magnetic plate 112 is inserted into the pump body 11 to continue the liquid transfer work. Because the pump body 11 is minimally invasively implanted in the shallow surface layer, the site of implanting the pump body 11 is found by positioning, and the magnetic plate 112 is minimally invasively extracted for MR examination. After the examination, the magnetic plate 112 is encapsulated in the pump body 11 for liquid transfer. Since the magnetism required in the first control valve 13, the second control valve 14 and the one-way valve 163 is small, they will not have much influence on the MR examination, so it is unnecessary to take them out.

In this embodiment, as shown in Fig. 1, the implantable liquid transfer device further includes a capsule 12, and one end of the liquid inlet pipe 15 far from the pump body 11 is connected with the capsule 12, and the capsule 12 is provided with a plurality of through holes 121. By providing the capsule 12, and a plurality of through holes 121 are provided on the capsule 12, large granular substances (such as macromolecular proteins, cell fragments and local soft tissue fragments) are prevented from entering the capsule 12, which avoids causing pipeline blockage.

Further, as shown in Fig. 5, the through hole 121 of the capsule 12 is provided with a semi-permeable membrane 121a, and body fluids can pass through the semi-permeable membrane 121a and enter the capsule 12. The semi-permeable membrane 121a can only allow liquid or small molecules to enter the capsule 12, further avoiding pipeline blockage. The capsule 12 is a flexible material with certain contraction or expansion. Under the drive of the pump body 11, when the capsule 12 contracts, the liquid in the edema area enters the capsule 12 and flows in the direction of the pump 11 and the liquid outlet pipe 16 through the liquid inlet pipe 15. Preferably, the capsule 12 can be made of biodegradable materials, and the capsule 12 can be implanted in the target area when drainage or monitoring physiological indexes are needed within a required time. When the treatment is completed, the capsule 12 can be biodegradable in body without been taken out, and no injury residue is generated.

As shown in Fig. 6, the capsule 12 has a plurality of branch structures 122, each branch structure 122 is respectively provided in a different edema area, and each branch structure 122 is provided with a plurality of through holes 121, and is communicated with the liquid inlet pipe 15. The plurality of branch structures are beneficial to improve the management effect of the accumulated liquid, and the merged port of each branch structure 122 is communicated with the liquid inlet pipe 15. Each branch structure 122 can be independently located in different tissues, such as lung, chest, brain, etc., and controlled by one pump body 11. The effusion of different tissues is sucked through the liquid inlet and transferred to the abdominal cavity for absorption. It is also possible to design a control valve on each branch structure 122 to control each branch separately. Sensors can be installed in each branch structure 122 to monitor the transfer demand and physiological conditions of each area.

Further, both the first control valve 13 and the second control valve 14 are magnetic control valves, and both the first control valve 13 and the second control valve 14 can change the conduction state by magnetic force, so as to control the normal operation of the first control valve 13 and the second control valve 14 by magnetic field outside the body.

In this embodiment, as shown in Fig. 3, the first control valve 13 includes a first valve housing 131, a first flexible pipe 132 and a first magnetic rotation member 133. The first valve housing 131 has a disc-shaped structure as a whole, and the first valve housing 131 has a first accommodating cavity 131a and two second ports 131b communicating with the first flexible pipe 132. The first magnetic rotation member 133 is eccentrically provided (i.e., the axis of rotation is not at the same position as the center of the first magnetic rotation member 133) in the first accommodating cavity 131a and can rotate in the first accommodating cavity 131a, and the first magnetic rotation member 133 can rotate in a magnetic field and adjust the flow of the first flexible pipe 132. The first magnetic rotation member 133 rotates with the drive of the external magnetic field, and the first flexible pipe 132 is squeezed differently during the rotation. Within a certain range, liquid can be allowed to pass through the liquid inlet pipe 15, and the first magnetic rotation member 133 is continued until no liquid passes through the first flexible pipe 132, which is in a closed state, so that the flow rate in the transfer process can be adjusted and a safe and reliable transfer can be realized.

In this embodiment, as shown in Fig. 4, the second control valve 14 includes a second valve housing 141 and a second magnetic rotation member 142. The second valve housing 141 has a second accommodating cavity 141a and three third ports 141b communicating with the second accommodating cavity 141a, and the three third ports 141b communicate with the liquid outlet pipe 16, the first branch pipe 161 and the second branch pipe 162 respectively. The second magnetic rotation member 142 is provided in the second accommodating cavity 141a and forms a liquid guide channel 141c with an inner wall of the second valve housing 141. The second magnetic rotation member 142 can rotate in the second accommodating cavity 141a. The second magnetic rotation member 142 includes a rotation part 142a and a blocking part 142b which are connected with each other. The second magnetic rotation member 142 can rotate in a magnetic field and enable the blocking part 142b to selectively block one of the third ports 141b. At least one of the rotation part 142a and the blocking part 142b is magnetic, the rotation part 142a is made of biocompatible elastic material, and the blocking part 142b is a spherical structure. Only when the blocking part 142b rotates to the corresponding third port 141b, the third port 141b is closed by the blocking part 142b. Of course, the blocking part 142b can rotate independently, that is, the blocking part 142b is made of magnetic material. In other embodiments, when the end of the outlet pipe 16 far away from the pump body 11 can be provided with more branch pipes, the number of the blocking parts 142b can be correspondingly increased according to actual application scenarios.

Further, the one-way valve 163 includes a duckbill valve, a diaphragm valve, a ball valve or a contraction and expansion pipe valve.

In this embodiment, as shown in Fig. 8, the one-way valve 163 is a diaphragm valve, and the diaphragm valve includes a valve pipe 163a and a plurality of magnetic membrane flaps 163b provided in the valve pipe 163a. The magnetic membrane flaps 163b can be unfolded or rolled up in a magnetic field. In an initial state, the magnetic membrane flaps 163b curl in the valve pipe 163a and block the conduction of the valve pipe 163a. In an infusion state, the magnetic membrane flaps 163b expands and curls at intervals in the infusion direction, that is, when the next magnetic membrane flap 163b expands, the last magnetic membrane flap 163b curls. The magnetic membrane flap 163b can be made of flexible materials with magnetism, such as silica gel doped with magnetic powder, or a composite material formed by magnetic powder and polymer flexible materials. The magnetic membrane flap 163b is curled without external driving, and the curling of the magnetic membrane flap 163b can be accelerated by applying a reverse magnetic field. Stimulated by the external magnetic field, the curl state changes to the extended state, and the contracted state changes to the open state, so as to realize an oriented flow of liquid.

As shown in Fig. 8, when no liquid passes through, the magnetic membrane flap 163b is curled. As shown in Figs. 9a-9d, when the liquid enters, the first magnetic membrane flap 163b assumes an extended structure under the control of the external magnetic field, and the liquid enters between the first magnetic membrane flap 163b and the second magnetic membrane flap 163b. At this time, the first magnetic membrane flap 163b is closed and the second magnetic membrane flap 163b is opened, and with the flow of the liquid, the second magnetic membrane flap 163b is closed and the third magnetic membrane flap 163b is opened, which in turn ensures the closing sequence and operation time of each magnetic membrane flap 163b, and realizes an oriented flow of liquid..

In this embodiment, as shown in Figs. 1 and 7, the fixing device 164 includes an injection port 164a, a control pipe 164b and a plurality of microbubble structures 164c, and the injection port 164a communicates with the plurality of microbubble structures 164c through the control pipe 164b. The microbubble structure 164c is made of elastic materials, such as flexible polymer materials, such as polyurethane and silica gel, which have certain elastic deformation. The outer surface of the microbubble structure 164c is designed with a coating, such as heparin, to enhance histocompatibility and facilitate internal fixation. Liquid or gas is injected into the microbubble structure 164c through the injection port 164a, so that the microbubble structure 164c expands and fixes the first branch pipe 161 to the target tissue, such as the bladder 30 or vein, thereby avoiding sloshing in the tissue and liquid environment and affecting the liquid transfer. The fixing device 164 can be integrated with the first branch pipe 161, and the channels of the control pipe 164b and the first branch pipe 161 are separated from each other.

In this embodiment, the implantable liquid transfer device 10 further includes a data monitoring component 102, which is used for monitoring body fluid information, and the data monitoring component 102 includes at least one of a pressure sensor, a flow sensor or an inflammatory factor sensor, wherein the pressure sensor is used for detecting the hydraulic pressure of the edema area, and the flow sensor is used for detecting the flow of the liquid transferred by the pump body 11. When the pressure in the edema area reaches a certain limit, the pump body 11, the first control valve 13 and the second control valve 14 are opened; and with the continuous transfer of liquid, the pressure in the target area decreases, and when the pressure is lower than the expected set value, the pump body 11 and the first control valve 13 are closed, which ensures the safety during transfer, and avoid adverse events such as local tissue low pressure and electrolyte disorder caused by the excessive transfer volume at one time. When the flow sensor detects that the pump body 11 has completed the target transfer volume, the valve is closed, otherwise, the valve is opened. The inflammatory factor sensor is used to detect the inflammatory condition in the edema area. If the inflammation is too serious, the communication module 25 is used to send body fluid information to the mobile terminal to remind patients and medical staff.

The present application also provides a liquid transfer control system. As shown in Fig. 10, the liquid transfer control system includes a transfer control device 20 and the implantable liquid transfer device 10 as described above, and the transfer control device 20 is used to control the normal operation of the implantable liquid transfer device 10. The transfer control device 20 can be located outside the body or inside the body, preferably outside the body, and controls the normal operation of the implantable liquid transfer device 10 through the wireless transmission function. The implantable liquid transfer device 10 has no electronic active parts, and is simple in structure and convenient for the operation of the control system.

In this embodiment, the transfer control device 20 includes a power supply 21, a processor 22, a driving module 23 and a data receiving module 24, and the power supply 21, the driving module 23 and the data receiving module 24 are electrically connected to the processor 22. The power supply 21 is used for supplying electric energy to the transfer control device 20; The driving module 23 is used to drive the implantable liquid transfer device 10 to operate normally. The data receiving module 24 is used for receiving data signals of the implantable liquid transfer device 10; The processor 22 processes the body fluid information and controls the normal operation of the whole transfer control device 20.

Further, the implantable liquid transfer device 10 includes a pump body 11, a control valve 101 and a data monitoring module 102, wherein the driving module 23 is used for driving the pump body 11, the control valve 101 and the data monitoring module 102 to operate; and the data monitoring module 102 is used for monitoring body fluid information; and the data receiving module 24 is used for receiving body fluid information; and the processor 22 controls the operation state of the driving module 23 to drive the pump body 11 and the control valve 101 according to the body fluid information.

The control valve 101 includes a first control valve 13, a second control valve 14 and a one-way valve 163. The data monitoring module 102 includes a pressure sensor, a flow sensor and an inflammatory factor sensor. The pressure sensor is used to detect the hydraulic information of the edema area, the flow sensor is used to detect the flow information of the liquid transferred by the pump body 11, and the inflammatory factor sensor is used to detect the inflammatory information of the edema area. The processor 22 controls the driving module 23 to drive the pump body 11 and the control valve 101 according to the hydraulic information, the flow information and the inflammatory information. The pressure sensor and the flow sensor can be provided in the pump body 11, the liquid inlet pipe 15 and the liquid outlet pipe 16, and the inflammatory factor sensor can be provided on the capsule 12 to monitor the inflammatory condition at the tissue edema.

A plurality of driving modules 23 can be provided and correspond to the pump body 11, the first control valve 13, the second control valve 14, the one-way valve 163, the pressure sensor, the flow sensor and the inflammatory factor sensor respectively, so as to ensure that the driving modules 23 are coupled with the pump body 11, the control valve 101 and the data monitoring component 102 within a certain range. The driving module 23 controls the normal operation of the pump body 11, the first control valve 13, the second control valve 14 and the one-way valve 163 through magnetic force, and provides electric energy to the pressure sensor, the flow sensor and the inflammatory factor sensor through electromagnetic induction. The data receiving modules 24 may also be multiple and correspond to the pressure sensor, the flow sensor and the inflammatory factor sensor respectively, and receive the body fluid information detected by the pressure sensor, the flow sensor and the inflammatory factor sensor through the wireless receiving function.

Further, the transfer control device 20 further includes a communication module 25 and a memory 26, both of which are electrically connected with the processor 22, and the communication module 25 is used for sending the body fluid information monitored by the implantable liquid transfer device 10 to the mobile terminal; The memory 26 is used to store control programs and body fluid information.

The liquid transfer control system provided by the present application converts passive transfer into active transfer, manages the transfer of hydrops in various tissues, and can realize the functions of monitoring, drug administration, sampling detection, etc., and can be applied to various transfer scenarios, and can be applied to various tissues and symptoms in vivo, such as drainage of cerebrospinal fluid, drainage of ascites in abdominal cavity, drainage during glaucoma surgery, drainage of lymph in limbs caused by lymphedema, etc. By controlling the normal operation of the implantable liquid transfer device 10 through the transfer control device 20 and ensuring that the transfer control device 20 and the implantable liquid transfer device 10 are coupled within a certain range, remote driving can be realized. The safety and reliability of transferring body fluids or local tissue fluids is realized.

In the present application, the locative words such as up, down, left, right, front and back are defined by the positions of the structures in the drawings and the positions between the structures, which are used to express the clarity of the technical solution and convenience. It should be understood that the use of the locative words should not limit the scope of the present application. It should also be understood that the terms "first" and "second" used in the present application are only used to distinguish names, and are not used to limit the number and order.

The above is only preferred embodiments of the present application, and does not limit the present application in any form. Although the present application has been disclosed in preferred embodiments as above, preferred embodiments are not used to limit the present application. Any skills familiar with this filed can make some changes or modifications by using the technical contents disclosed above without departing from the scope of the technical solution of the present application, which is an equivalent embodiment with equivalent changes. However, any simple modifications, equivalent changes and modifications made to the above embodiments according to the technical essence of the present application without departing from the technical solution of the present application still fall within the protection scope of the present application.

### Industrial practicability

By providing the pump body in an implantable liquid transfer device, the pump body is implanted in the body together with the implantable liquid transfer device, so that the liquid can be actively transferred to the natural cavity to be discharged, the balance of local tissue pressure is maintained, the infection caused by repeated punctures is avoided, and the pump body has good universality for ascites patients; The design of multiple branch pipes can be used to transfer liquids, administer drugs or take samples for testing, and drain the liquids which are formed by such as malignancy of a tumor and containing inflammatory factors to the outside of the body, so as to realize the multifunctional transfer of liquid management.

## Claims

1. An implantable liquid transfer device, comprising a pump body (11), a control valve (101), a liquid inlet pipe (15) and a liquid outlet pipe (16), wherein the control valve (101) comprises a first control valve (13) and a second control valve (14), and one end of the liquid inlet pipe (15) is connected with one end of the pump body (11), and one end of the liquid outlet pipe (16) is connected with the other end of the pump body (11), and the pump body (11) is configured to drive body fluid to flow from the liquid inlet pipe (15) to the liquid outlet pipe (16), and the other end of the liquid outlet pipe (16) is provided with a plurality of branch pipes (160); and the first control valve (13) is provided on the liquid inlet pipe (15); and the second control valve (14) is provided on the liquid outlet pipe (16), and the second control valve (14) is a multi-way valve and is configured to control the liquid outlet pipe (16) to be communicated with the branch pipes (160), and the plurality of the branch pipes (160) are respectively provided at different tissues.

2. The implantable liquid transfer device according to claim 1, wherein the pump body (11) is a positive displacement pump, and the positive displacement pump comprises a pump housing (111) and a magnetic plate (112) connected with the pump housing (111), wherein the pump housing (111) has a cavity (111a), and the magnetic plate (112) is provided in the pump housing (111) and divides the cavity (111a) into two chambers (111c), and the pump housing (111) is provided with two first ports (111b) communicating with one of the chambers (111c), and the magnetic plate (112) is movable or deformable in a magnetic field and changes the volumes of the two chambers (111c).

3. The implantable liquid transfer device according to claim 2, wherein an edge of the magnetic plate (112) is provided with a clamping block (113), and the magnetic plate (112) is clamped with the pump housing (111) through the clamping block (113).

4. The implantable liquid transfer device according to claim 2, wherein the two first ports (111b) are detachably connected with the liquid inlet pipe (15) and the liquid outlet pipe (16) respectively.

5. The implantable liquid transfer device according to claim 1, wherein the implantable liquid transfer device further comprises a capsule (12), and one end of the liquid inlet pipe (15) far away from the pump body (11) is connected with the capsule (12), and the capsule (12) is provided with a plurality of through holes (121).

6. The implantable liquid transfer device according to claim 5, wherein a semi-permeable membrane (121a) is provided at the through holes (121) of the capsule (12), and body fluids can pass through the semi-permeable membrane (121a) and enter the capsule (12).

7. The implantable liquid transfer device according to claim 5, wherein the capsule (12) has a plurality of branch structures (122), wherein every branch structure (122) is respectively provided in a different edema area, and each branch structure (122) is provided with a plurality of through holes (121), and each branch structure (122) is connected with the liquid inlet pipe (15).

8. The implantable liquid transfer device according to claim 1, wherein the first control valve (13) is a magnetic control valve, and the first control valve (13) comprises a first valve housing (131), a first flexible pipe (132) and a first magnetic rotation member (133), and the first valve housing (131) has a first accommodating cavity (131a) and two second ports (131b) communicated with the first flexible pipe (132), and the first magnetic rotation member (133) is eccentrically provided in the first accommodating cavity (131a) and can rotate in the first accommodating cavity (131a), and the first magnetic rotation member (133) can rotate in a magnetic field and adjust the flow of the first flexible pipe (132).

9. The implantable liquid transfer device according to claim 1, wherein one end of the liquid outlet pipe (16) far away from the pump body (11) is provided with two branch pipes (160), and the two branch pipes (160) are a first branch pipe (161) and a second branch pipe (162) respectively, and the control valve (101) further comprises a one-way valve (163), and the first branch pipe (161) is provided with the one-way valve (163) and a fixing device (164), and the fixing device (164) is provided at one end of the first branch pipe (161) far away from the liquid outlet pipe (16); and the second branch pipe (162) is provided with a fixing point (165)

10. The implantable liquid transfer device according to claim 9, wherein the second control valve (14) is a magnetic control valve, and the second control valve (14) comprises a second valve housing (141) and a second magnetic rotation member (142), and the second valve housing (141) has a second accommodating cavity (141a) and three third ports (141b) communicating with the second accommodating cavity (141a), and the three third ports (141b) are respectively connected with the liquid outlet pipe (16), the first branch pipe (161) and the second branch pipe (162), and the second magnetic rotation member (142) is provided in the second accommodating cavity (141a) and forms a liquid guide channel (141c) with an inner wall of the second valve housing (141), and the second magnetic rotation member (142) can rotate in the second accommodating cavity (141a), and the second magnetic rotation member (142) comprises a rotation part (142a) and a blocking part (142b) which are connected with each other, and the second magnetic rotation member (142) can rotate in a magnetic field and enable the blocking part (142b) to selectively block one of the third ports (141b).

11. The implantable liquid transfer device according to claim 9, wherein the one-way valve (163) is a diaphragm valve, and the diaphragm valve comprises a valve pipe (163a) and a plurality of magnetic membrane flaps (163b) provided in the valve pipe (163a), and the magnetic membrane flaps (163b) can be unfolded or rolled up in a magnetic field; wherein in an initial state, the magnetic membrane flap (163b) curls in the valve pipe (163a) and blocks the conduction of the valve pipe (163a); and in an infusion state, the magnetic membrane flap (163b) unfolds and curls at intervals in an infusion direction.

12. The implantable liquid transfer device according to claim 9, wherein the fixing device (164) comprises an injection port (164a), a control pipe (164b) and a plurality of microbubble structures (164c), and the injection port (164a) is communicated with a plurality of microbubble structures (164c) through the control pipe (164b), and the microbubble structures (164c) are made of elastic materials.

13. The implantable liquid transfer device according to claim 1, wherein the implantable liquid transfer device (10) further comprises a data monitoring component (102) for monitoring body fluid information, and the data monitoring component (102) comprises at least one of a pressure sensor, a flow sensor or an inflammatory factor sensor, wherein the pressure sensor is configured to detect a hydraulic pressure of the edema area, the flow sensor is configured to detect a flow of the liquid transferred by the pump body (11), and the inflammatory factor sensor is configured to detect the inflammatory condition of the edema area.

14. A liquid transfer control system, comprising a transfer control device (20) and the implantable liquid transfer device (10) according to any one of claims 1-13, wherein the transfer control device (20) is configured to control a normal operation of the implantable liquid transfer device (10).

15. The liquid transfer control system according to claim 14, wherein the transfer control device (20) comprises a power supply (21), a processor (22), a driving module (23) and a data receiving module (24), and the power supply (21), the driving module (23) and the data receiving module (24) are all electrically coupled with the processor (22), and the implantable liquid transfer device (10) comprises a data monitoring component (102), and the driving module (23) is configured to drive the pump body (11), the control valve (101) and the data monitoring component (102) to operate, and the data monitoring component (102) is configured to monitor body fluid information, and the data receiving module (24) is configured to receive the body fluid information, and the processor (22) controls the operation state of the driving module (23) for driving the pump body (11) and the control valve (101) according to the body fluid information.

16. The liquid transfer control system according to claim 15, wherein the transfer control device (20) further comprises a communication module (25), which is configured to send the body fluid information to a mobile terminal.
